# EUROPEAN PATENT APPLICATION

(11) **EP 1 016 865 A2**
(43) Date of publication of application: **05.07.2000**
(21) Application number: 99310246.6
(22) Date of filing: 20.12.1999
(51) Int. Cl.: G01N 33/50

(54) **Macro-complexed ligand binders**

(30) Priority: 21.12.1998 GB 9828192
(71) Applicant: Ortho-Clinical Diagnostics, Amersham, Buckinghamshire HP7 OHJ (GB)
(72) Inventor: Dawkes, Adrian Charles, Windsor, Berkshire SL4 3PF (GB)
(74) Representative: Mercer, Christopher Paul

(57) **Abstract**

There is described a macro-complex formed by attaching one or more biotinylated ligand binder molecules via streptavidin to one or more biotinylated carrier molecules, the macro-complex having more free biotin moieties than ligand binding sites and capable of being immobilised onto a solid support, and a method for performing assays using it.

## Description

### Background of the invention

The detection and measurement of biological and chemical entities in body fluids is used in the diagnosis and monitoring of a wide range of clinical conditions. It is desirable to provide appropriate clinical information in relatively short times. It is therefore important that assays providing information of this nature can be completed within minutes.

Measurements based on the use of binding reactions between the analyte and one or more specific binders are well known and have been used for many years. The two components of a specific binding reaction are often referred to as the ligand and the ligand binder, or as the analyte and the analyte blinder when the ligand is the analyte being measured. The two components are capable of binding specifically to each other and form the basis of a range of analytical methods. In many assays using a specific binding reaction, the analyte or the analyte binder is labelled with a detectable group. In a competitive binding assay, the labelled analyte competes with the analyte in the sample for binding to an analyte binder. In other types of binding assays, a labelled analyte binder is used to react with the analyte in the sample and with a second analyte binder. In both types of assay the labelled reactant becomes bound to the unlabelled analyte binder in an analyte concentration-dependent manner. Measurement of the labelled reactant in this complex gives an estimate of the concentration of analyte in the sample.

Solid phase assays, in which the labelled reactant becomes bound to an analyte binder immobilised on a solid surface during the assay, allow easy separation of the labelled reactant which is bound to its specific binder from the labelled form which is not bound. In solid phase assays, the amount of a labelled reactant which is either bound to the solid phase or which is in the liquid phase gives a measure of the amount of analyte in the sample.

Modification of analyte binders by the attachment of an unrelated ligand in order to achieve reversible attachment of the analyte binder to a solid phase is described in US Patent No. 4,271,140.

Further improvements in the level of functionality of bioactive surfaces have been made using biotin-streptavidin and biotin-avidin linkages where a biotinylated ligand binder is captured by avidin or streptavidin immobilised on a solid phase. These improvements are well documented (Butler et al., 1992; Davies et al., 1993; Davies et al., 1994.).

The use of a complex in which the analyte binder is chemically coupled to a soluble matrix or backbone which carries a ligand is described in US Patent No. 4,778,751. The ligand is used to immobilise the complex on to a solid phase during the assay. The advantages of using a complex of this structure are that the active components are chemically coupled together, and that the number of immunocomplexes which can be immobilised on a solid support is increased. This is due to the high ratio of analyte binder molecules in the complex compared to the number of ligand molecules in the complex. The use of a complex in which the active components are attached to each other through the use of biotin-streptavidin linkages is not disclosed. The problems of poor assay sensitivity and precision in competitive binding assays is not discussed.

In order to achieve rapid assays in which specific binding partners are utilised, that have appropriate dynamic range, sensitivity and clinical performance it is often desirable to limit the quantity of analyte binder in the system. This is particularly true in assays that rely on a competitive binding format between the analyte in the sample and labelled analyte. In systems that rely on solid phase capture of the analyte binder, equilibrium may not be achieved in the time available to do the assay. As a consequence, small variations in the quantity of analyte binder captured by the solid phase at the selected end point time of the assay can result in unacceptable variations in the detection of the labelled analyte bound to the analyte binder. This translates into poor reproducibility and sensitivity in the assays.

In assays using a biotinylated ligand binder, control of the quantity of biotinylated ligand binder that is ultimately captured by streptavidin immobilised on the solid phase can be difficult to achieve consistently, especially where limited time is available to complete the assay. Control of the capture of the biotinylated ligand binder is also difficult where the biotinylated ligand binder is required at low concentration (e.g. in competition binding assay formats) or if the ligand binder has a low number of biotin moieties coupled to it that are available to bind to the streptavidin. Thus, there remains a need for improvements in the efficiency and reproducibility of a ligand binder capture system that would give improved assay precision and hence improved clinical performance.

### Summary of Invention

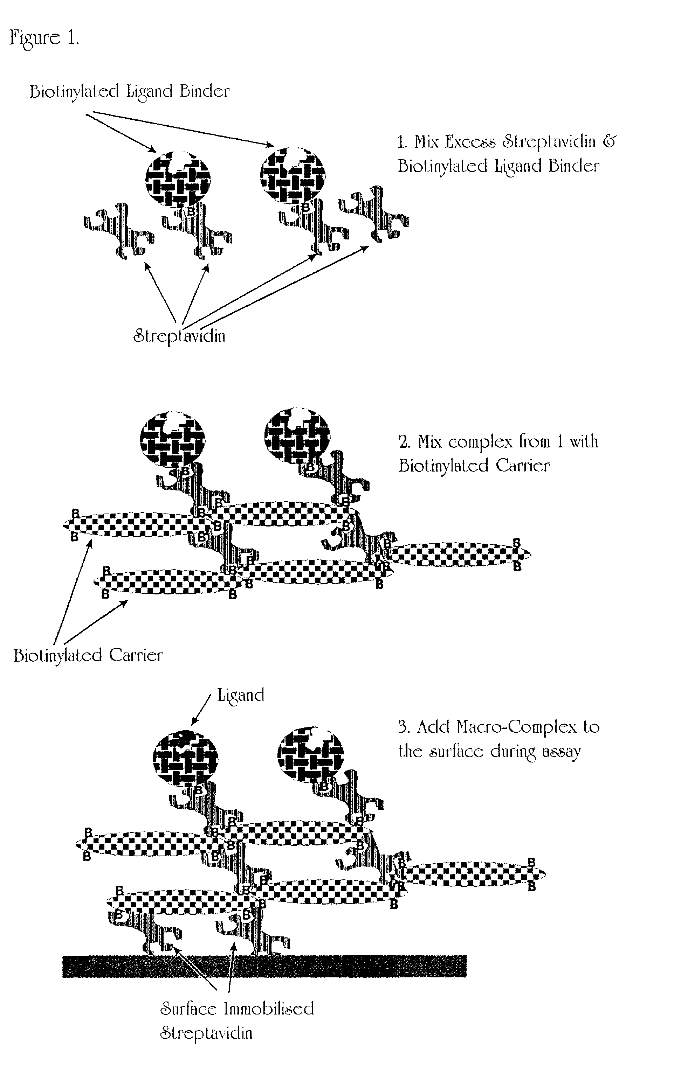

Applicants have achieved a means for improving assay precision and sensitivity with greater efficiency and reproducibility than conventional means. It is one objective of the present invention to provide a macro-complex useful for improving assay precision and sensitivity with greater efficiency and reproducibility by complexing biotinylated ligand binder (B-LB) to streptavidin (SAV) and subsequently complexing this to a biotinylated carrier molecule (B-CAR) that has multiple free (unbound) biotin moieties at the surface e.g. biotinylated bovine serum albumin (B-BSA). This macro-complex can then be presented in solution such that it contacts the immobilised streptavidin surface. The macro-complex can be preformed before performing the assay or can be formed *in situ.* A preferred embodiment is a macro-complex formed by attaching one or more ligand binder molecules to one or more carrier molecules via biotin-streptavidin linkages, the macro-complex having more free biotin moieties than ligand binding sites and capable of being immobilised onto a solid support (see Figure 1).

Another embodiment of the invention relates to a method for performing an assay using the macro-complex. A preferred embodiment relates to a method for performing an assay for vitamin B12 or folate using the macro-complex.

### Brief Description of the Drawings

- Figure 1:: A macro-complex of biotinylated ligand binder, biotinylated carrier, and streptavidin.
- Figure 2:: Dose response curve of a vitamin B12 assay showing improved precision and sensitivity.

### Detailed Description of the Invention

A "ligand binder" can be any molecule capable of specific binding to a ligand. Typically the ligand binder would be capable of binding an analyte of interest in a sample or to a labelled analogue thereof. The ligand binder typically would be a binding protein such as intrinsic factor, folate binding protein, a steroid binding protein or a specific receptor, or a fragment or derivative of any of the foregoing. A ligand binder can be the larger or the smaller of the two component parts of a specific binding pair, the other component being the ligand. For this invention the ligand binder can be either the larger or smaller component of the binding pair. A preferred ligand binder is intrinsic factor.

A "carrier" shall mean a molecule or polymer that can be attached directly or indirectly via biotin-streptavidin linkages to the ligand binder. A preferred carrier would be capable of having more than one biotin molecule attached to it. The carrier can be natural or synthetic. A preferred carrier is bovine serum albumin (BSA).

In order to facilitate the attachment of the ligand binder to the carrier, the ligand binder and carrier molecules can be modified by the chemical attachment of biotin. Methods for attaching biotin to other molecules are known in the art. See for example, Savage, M.D., 1996, An Introduction to Avidin-Biotin Technology and Options for Biotinylation. In A Laboratory Guide to Biotin-Labeling in Biomolecule Analysis. Eds. T. Meier & F. Fahrenholz. Pubs. Birkhäuser.

Methods for immobilising streptavidin to a solid support are known in the art. Coating techniques include, for example, chemical binding or physical adsorption (M. Suter and J.E. Butler, 1986, Immunology Letters, volume 13, pages 313-316).

It is within the skill of the ordinary artisan to select a suitable labelled analogue or labelled binder of an analyte to be measured. Suitable labels include radioactive isotopes, enzymes and fluorescent or chemiluminescent molecules and methods of labelling analyte molecules or binders are well known in the art. A preferred label is the enzyme horseradish peroxidase.

A solid support can be any support or surface, for example, a microwell, gel, membrane, particles, beads or a dipstick. One skilled in the art would know what type of support best suits the assay to be performed.

It is to be understood that the invention herein can equally employ biotin-avidin wherever biotin-streptavidin linkages are discussed. The skilled artisan will know which material is more suitable for a given situation. Likewise the macro-complex can be formed prior to performing the assay or *in situ.*

One embodiment of the present invention is where a low concentration of B-LB is complexed with a molar excess of SAV resulting in (B-LB)-(SAV)ₓ complexes. Addition of a molar excess of biotinylated carrier over SAV and allowing complexation results in polymerisation of the complexes to give (B-LB)-(SAV)ₓ-(B-CAR)_{y} macro-complexes of varying size and composition (see Figure 1). Large molecules are generated that have increased mass and multiple free biotins that can bind to the immobilised SAV. The efficiency and reproducibility of capture of these molecules compared to the uncomplexed B-LB is enhanced.

Increasing the mass of the functional analyte binding molecule whilst increasing the number of available biotins to bind to the SAV surface has a dual effect. The mass increase means that the complex is retained in the liquid phase for longer since it will take longer to diffuse to the surface. This increases the relative amount of time that the assay is conducted in the liquid phase compared to the liquid/solid phase interaction. In this instance the reaction kinetics of analyte binding to the B-LB are more favorable than those of analyte binding to surface immobilised B-LB. Once the complexes do diffuse to the surface there is an increased probability of an interaction with the surface that results in capture via biotin-streptavidin linkages due to the increased number of biotins on the macro-complex. The overall assay reaction time does not appear to be significantly affected by the use of a macro-complex.

The above described complex is believed to be more advantageous than a ligand binder that has been chemically linked to a carrier molecule even though the same increase in mass and number of available biotin molecules could be achieved. By "chemically linked" it is meant that the ligand binder and the carrier are attached to each other via covalent bonds and not via specific binding reactions. Specific binding molecules can be chemically linked to each other using activated esters and heterobifunctional reagents or other reagents which are well known in the art. A problem encountered when using chemical linking is that linking is not reproducible either in terms of the number of ligand binder molecules chemically linked to the carrier, or with respect to the proportion of ligand binder molecules remaining functional after linking.

Determination of the amount of ligand binder in a complex after chemical linking is extremely difficult. Biological components are often analysed by spectroscopic means, and if the ligand binder and other components of the complex are of similar material, e.g. proteinaceous, it is difficult to distinguish the ligand binder from the other components in the complex. Addition of the correct activity of ligand binder to the assay can then only be achieved by titration. This process is prone to error and can give differences in activity of the ligand binder in the assay on different occasions. This results in poorer reproducibility of the assay. The generation of macro-complexes by the (B-LB)-(SAV)ₓ-(B-CAR)_{y} route permits control over the concentration and activity of each individual component of the macro-complex. Unexpectedly it was found that a macro-complex containing a high ratio of free biotin moieties to ligand binder sites gave a more reproducible immobilisation of the macro-complex onto a streptavidin solid phase. A preferred macro-complex contains a molar ratio of about 1 part of biotinylated intrinsic factor to 16 parts of streptavidin to 28 parts of biotinylated bovine serum albumin.

### Example 1

### Vitamin B12 Assay on Streptavidin Coated Microwells using Macro-Complexed Biotinylated Intrinsic Factor (New Method) or Biotinylated Intrinsic Factor (Existing Method)

### I) Preparation of Biotinylated Intrinsic Factor Macro-complexes for use in a Vitamin B12 assay.

1. The diluent buffer used was 0.1M sodium phosphate in deionised water, pH 7.0, containing 0.01% weight per volume (w/v) potassium cyanide, 0.5% (w/v) bovine serum albumin (BSA) and 0.5% volume per volume (v/v) BRONIDOX-L as a preservative.
2. Intrinsic factor was biotinylated using biotin-LC-NHS (Pierce and Warriner, Chester, U.K.) and bovine serum albumin (BSA) was biotinylated using biotin-XX-NHS (Calbiochem, Beeston, U.K.) by methods described in Savage, M.D., 1996 - see above.
3. Biotinylated intrinsic factor (B-IF) and streptavidin were mixed at 24 ng/ml of B-IF with 0.5 µg/ml streptavidin in the diluent buffer. This was left to equilibrate for at least 18 hours at 2-8°C.
4. Biotinylated BSA was added at 1.0 µg/ml to the (B-IF)-streptavidin complexes and incubated for 1 hour at room temperature.

The comparative reagent (existing method) was uncomplexed biotinylated intrinsic factor (B-IF) at 24 ng/ml in the diluent buffer.

### II) Vitamin B12 Assay Protocol

1. To a test tube was added:
   - 150 µl sample or standard serum containing vitamin B12
   - 50 µl Stabiliser (1.6% w/v dithiothreitol in deionised water)
   - 100 µl Denaturant (0.85M sodium hydroxide in deionised water containing 0.01% w/v potassium cyanide, 0.15M disodium tetraborate, 0.01% w/v Mordant Orange dye)
2. The solution was mixed and incubated at room temperature for 15 min.
3. 100 µl Neutraliser (0.6M boric acid in deionised water containing 30mM potassium iodate, 2.5% v/v TRITON X-100, 0.01% w/v Medical antifoam C) was added.
4. The resulting solution was mixed again.
5. 60 µl of the solution was transferred to a VITROS ECi streptavidin coated microwell (manufactured by Ortho-Clinical Diagnostics)
6. 50 µl of a 0.1M potassium phosphate buffer in deionised water, pH 6.0, containing a vitamin B12-horseradish peroxidase conjugate, 0.5% (w/v) human serum albumin, 5% (w/v) sucrose, 0.5% (w/v) chloroacetamide as a preservative and 0.05% Medical antifoam C was added. The vitamin B12-horseradish peroxidase conjugate was prepared according to the method in US Patent No. 4,465,775 using a mixture of vitamin B12 monocarboxylic acid isomers.
7. 50µl of B-IF or B-IF Macro-complex reagent prepared as above was also added.
8. The mixture was then incubated for 30 min at 37°C
9. The microwells were then washed using VITROS ECi Wash Reagent and 100 µl of each part of VITROS ECi Signal Reagent was added.
10. The light emitted from each microwell was the measured using a VITROS ECi Analyzer (Ortho-Clinical Diagnostics).

During the assay incubation the vitamin B12-horseradish peroxidase conjugate is bound by B-IF (existing method) or by B-IF in the macro-complex (new method) which becomes immobilised on the surface of the microwell by the reaction of the biotin on the biotinylated intrinsic factor with streptavidin on the well (existing method) or biotin on the macro-complex with streptavidin on the well (new method). After watching the microwells the activity of the immobilised vitamin B12-horseradish peroxidase complex is measured using an enhanced luminescence reaction (US Patent No. 5, 372, 931). VITROS ECi Signal Reagent, contains luminogenic substrates (a luminol derivative and a peracid salt) and an electron transfer agent. The horseradish peroxidase in the bound conjugate catalyzes the oxidation of the luminol derivative, producing light. The electron transfer agent (a substituted acetanilide) increases the level of light produced and prolongs its emission.

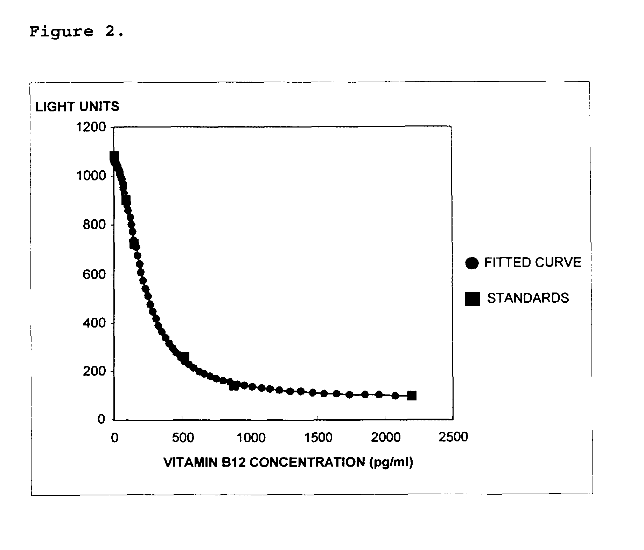

Figure 2 shows a dose response curve for the vitamin B12 assay over the range 0 pg/ml to 2000 pg/ml using the new method.

### Example 2

### Sensitivity of the vitamin B12 assay

The sensitivity of the vitamin B 12 assay is the concentration of vitamin B12 equivalent to the mean light signal of the 0 pg/ml vitamin B12 standard minus two times the standard deviation calculated using 20 replicates of the 0 pg/ml vitamin B12 standard. The sensitivity of the vitamin B12 assay using the existing method and the new method are shown in Table 1.

**Table 1**

| B-IF Type | Mean Light Units 0 pg/ml Vitamin B 12 | Light Units CV (%) | Sensitivity (pg/ml) | Serum Sample Mean Vitamin B 12 (pg/ml) | Vitamin B12 CV (%) |
|---|---|---|---|---|---|
| B-IF | 1078 | 5.2 | 62.5 | 450 | 11.5 |
| Macro-complex | 1056 | 1.6 | 14.9 | 510 | 2.9 |

It can be seen that the sensitivity of the existing method was 62.5 pg/ml. The sensitivity using the new method was 14.9 pg/ml. The improved reproducibility of the immobilisation of the macro-complexes on to the solid phase in the new vitamin B12 assay results in improved sensitivity of detection of vitamin B12.

### Example 3

### Precision of the vitamin B 12 assay

A serum sample containing a normal level of vitamin B 12 was assayed 20 times using the existing method and 20 times using the new method. The results are also shown in Table 1. The mean level of vitamin B12 was found to be 450 pg/ml in the existing method and 510 pg/ml in the new method. The coefficient of variation (CV%) of the measurement of vitamin B12 in the serum sample was 11.5% using the existing method and 2.9% in the new method. The improved reproducibility of the immobilisation of the macro-complexes on to the solid phase in the new vitamin B12 assay results in improved precision of measurement of vitamin B12 in the assay.

In conclusion, the instant macro-complex methodology shows improved assay sensitivity and precision. Accordingly, it should be noted that the present invention includes all modifications falling within the scope of the following claims.

## Claims

1. A macro-complex formed by attaching one or more biotinylated ligand binder molecules via streptavidin to one or more biotinylated carrier molecules, the macro-complex having more free biotin moieties than ligand binding sites and capable of being immobilised onto a solid support.

2. The macro-complex of claim 1 wherein the biotinylated ligand binder is intrinsic factor that has been biotinylated and the biotinylated carrier is bovine serum albumin that has been biotinylated and the biotinylated intrinsic factor and the biotinylated bovine serum albumin are attached to one another through streptavidin.

3. The macro-complex of claim 1 wherein the biotinylated ligand binder is folate binding protein that has been biotinylated and the biotinylated carrier is bovine serum albumin that has been biotinylated and the biotinylated folate binding protein and the biotinylated bovine serum albumin are attached to one another through streptavidin.

4. A method for performing an assay using the macro-complex of claim 1.

5. A method for performing an assay for vitamin B12 using the macro-complex of claim 2.

6. A method for performing an assay for folate using the macro-complex of claim 3.
